(19) Europäisches Patentamt
European Patent Office
Office européen des brevets

(11) EP 2 322 171 A2

(12) **EUROPEAN PATENT APPLICATION**

(43) Date of publication:
**18.05.2011 Bulletin 2011/20**

(21) Application number: **10188919.4**

(22) Date of filing: **26.10.2010**

(51) Int Cl.:
***A61K 31/277*** *(2006.01)* ***A61K 31/44*** *(2006.01)*
***A61K 51/04*** *(2006.01)* ***C07B 59/00*** *(2006.01)*
***C07C 233/83*** *(2006.01)* ***C07D 213/81*** *(2006.01)*

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(30) Priority: **17.11.2009 EP 09075507**

(71) Applicant: **Bayer Schering Pharma Aktiengesellschaft**
**13353 Berlin (DE)**

(72) Inventors:
- **Schmitt-Willich, Heribert**
  **10551 Berlin (DE)**
- **Böhnke, Niels**
  **12157 Berlin (DE)**
- **Koglin, Norman**
  **12587 Berlin (DE)**
- **Berndt, Mathias**
  **12163 Berlin (DE)**
- **Friebe, Matthias**
  **13509 Berlin (DE)**
- **Müller, André**
  **13591 Berlin (DE)**

(54) **Fluorine labeled L-glutamic acid derivatives**

(57) This invention relates to fluorine labeled L-glutamic acid derivatives suitable for labeling or already labeled with $^{18}F$ or $^{19}F$, methods of preparing such compounds, compositions comprising such compounds, kits comprising such compounds or compositions and uses of such compounds, compositions or kits for diagnostic imaging.

EP 2 322 171 A2

## Description

### Field of Invention

This invention relates to fluorine labeled L-glutamic acid derivatives suitable for labeling or already labeled with $^{18}F$ or $^{19}F$, methods of preparing such compounds, compositions comprising such compounds, kits comprising such compounds or compositions and uses of such compounds, compositions or kits for diagnostic imaging.

### Background

The invention relates to the subject matter referred to in the claims, i.e. fluorine labeled L-glutamic acid derivatives of the general formulas I and/or II, their precursors of the formula III, their use and their preparation processes.

The early diagnosis of malignant tumor diseases plays an important role in the survival prognosis of a tumor patient. For this diagnosis, non-invasive diagnostic imaging methods are an important aid. In the last years, in particular the PET (Positron Emission Tomography) technology has been found to be particularly useful. The sensitivity and specificity of the PET technology depends essentially on the signal-giving substance (tracer) used and on its distribution in the body. In the hunt for suitable tracers, one tries to make use of certain properties of tumors which differentiate tumor tissue from healthy surrounding tissue. The preferred commercial isotope used for PET applications is $^{18}F$. Owing to the short half-life of less than 2 hours, $^{18}F$ is particularly demanding when it comes to the preparation of suitable tracers. This isotope does not allow for complicated long synthesis routes and purification procedures, since otherwise a considerable amount of the radioactivity of the isotope will already have faded away before the tracer can be used for diagnosis. Accordingly, it is frequently not possible to apply established synthesis routes for non-radioactive fluorinations to the synthesis of $^{18}F$ tracers. Furthermore, the high specific activity of $^{18}F$ (about 80 GBq/nmol) leads to very low substance amounts of [$^{18}F$]-fluoride for the tracer synthesis, which in turn requires an extreme excess of precursor, making the result of a radio synthesis strategy based on a non-radioactive fluorination reaction unpredictable.

FDG ([$^{18}F$]-2-Fluorodeoxyglucose)-PET is a widely accepted and frequently used auxiliary in the diagnosis and further clinical monitoring of tumor disorders. Malignant tumors compete with the host organism for glucose as nutrient supply (Warburg O., Über den Stoffwechsel der Carcinomzelle [The metabolism of the carcinoma cell], Biochem.Zeitschrift 1924; 152: 309-339; Kellof G., Progress and Promise of FDG-PET Imaging for Cancer Patient Management and Oncologic Drug Development, Clin. Cancer Res. 2005; 11 (8): 2785-2807). Compared to the surrounding cells of the normal tissue, tumor cells usually have an increased glucose metabolism. This is exploited when using fluorodeoxyglucose (FDG), a glucose derivative which is increasingly transported into the cells, where, however, it is metabolically captured as FDG 6-phosphate after phosphorylation ("Warburg effect"). Accordingly, $^{18}F$-labeled FDG is an effective tracer for detecting tumor disorders in patients using the PET technology. In the hunt for novel PET tracers, recently, amino acids have been employed increasingly for $^{18}F$ PET imaging (for example (review): Eur. J. Nucl. Med. Mol. Imaging May 2002; 29(5): 681-90). Here, some of the $^{18}F$-labeled amino acids are suitable for measuring the rate of protein synthesis, but most other derivatives are suitable for measuring the direct cellular uptake in the tumor. Known $^{18}F$-labeled amino acids are derived, for example, from tyrosine amino acids, phenylalanine amino acids, proline amino acids, asparagine amino acids and unnatural amino acids (for example J. Nucl. Med. 1991; 32: 1338-1346, J. Nucl. Med. 1996; 37: 320-325, J. Nucl. Med. 2001; 42: 752-754 and J. Nucl. Med. 1999; 40: 331-338). $^{18}F$-labeled homoglutamic acid derivatives are not known up to date.

The PET tracers currently used in tumor diagnosis have some undisputed disadvantages: thus, FDG is preferably accumulated in cells having an elevated glucose metabolism; however, under different pathological and physiological conditions, as also in elevated glucose metabolism in the cells and tissues involved, for example infection sites or wound healing (summarized in J. Nucl. Med. Technol. (2005), 33, 145-155). Frequently, it is still difficult to ascertain whether a lesion detected via FDG-PET is really of neoplastic origin or is the result of other physiological or pathological conditions of the tissue. Overall, the diagnosis by FDG-PET in oncology has a sensitivity of 84% and a specificity of 88% (Gambhir et al., "A tabulated summary of the FDG PET literature", J. Nucl. Med. 2001, 42, 1-93S). The imaging of brain tumors, for example, is very difficult owing to the high accumulation of FDG in healthy brain tissue.

In some cases, the $^{18}F$-labeled amino acid derivatives currently known are well suited for the detection of tumors in the brain ((review): Eur. J. Nucl. Med. Mol. Imaging. 2002 May; 29(5): 681-90); however, in the case of other tumors, they are not able to compete with the imaging properties of the "Goldstandard" [$^{18}F$]2-FDG. The metabolic accumulation and retention of the current F-18-labeled amino acids in tumor tissue is generally lower than of FDG. In addition, the preparation of isomerically pure F-18-labeled non-aromatic amino acids is chemically very demanding.

Similarly to glucose, for glutamic acid and glutamine, too, an increased metabolism in proliferating tumor cells has been described (Medina, J. Nutr. 1131: 2539S-2542S, 2001; Souba, Ann Surg 218: 715-728, 1993). The increased rate of protein and nucleic acid syntheses and the energy generation per se are thought to be the reasons for an increased glutamine consumption of tumor cells. The synthesis of corresponding C-11- and C-14- labeled compounds, which are

thus identical to the natural substrate, has already been described in the literature (for example Antoni, Enzyme Catalyzed Synthesis of L-[4-C-11]aspartate and L-[5-C-11]glutamate. J. Labeled Compd. Radiopharm. 44; (4) 2001: 287-294 and Buchanan, The biosynthesis of showdomycin: studies with stable isotopes and the determination of principal precursors, J. Chem. Soc. Chem. Commun.; EN; 22; 1984; 1515-1517). First tests with the C-11-labeled compound indicate no significant accumulation in tumors.

It is an object of the present invention to provide novel compounds which, in [$^{18}$F]-labeled form, are suitable for PET-based diagnosis.

## Summary

The invention relates to the subject matter referred to in the claims, i.e. fluorine labeled L-glutamic acid derivatives of the general formulas I and/or II, their precursors of the formula III, their use and their preparation processes.

## Figure

Figure 1:

Examination of biological activity of compounds from the present invention in a cell-competition-experiment. (NCI-H460 non-small cell lung cancer cells, 10 min incubation with 1 µCi 3H-Glutamic acid in PBS-Puffer, concentration of competitor 1 mM).

## Description

In a first aspect, the invention is directed to compounds of the general formula (I)

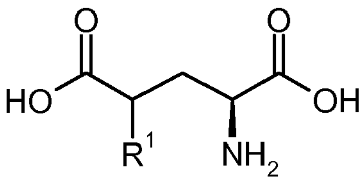

(I)

wherein $R^1$ is
F-aryl-E-A
wherein E is CO, CO-NH or NH-CO
and A is $C_1$-$C_6$ alkyl wherein the carbon chain may be interrupted by one oxygen atom with the proviso that there are at least two carbon atoms between two hetero atoms and
wherein the aryl moiety is a mono- or bicyclic aryl optionally substituted by 1 or 2 substituents independently selected from halogen, $NO_2$, CN, COOH, ($C_1$-$C_3$)alkyl, formyl, acetyl, alkoxycarbonyl or trifluoromethyl;
or
F-heteroaryl-E-A
wherein the heteroaryl moiety is a mono- or bicyclic heteroaryl wherein heteroaryl comprises 5 to 10 ring atoms wherein 1 or 2 ring atoms are independently selected from N, O or S and wherein the heteroaryl moiety is optionally substituted by 1 or 2 methyl groups.

Formula I encompasses single isomers, diastereomers and enantiomers, mixtures thereof and suitable salts thereof.

In a **first** embodiment, the invention is directed to a compound of general formula (I) wherein the Fluorine atom (F) is $^{18}$F isotope.

In a **second** embodiment, the invention is directed to a compound of general formula (I) wherein the Fluorine atom (F) is $^{19}$F isotope.

Preferably, Fluorine atom (F) is an $^{18}$F or $^{19}$F isotope.

Preferably, $C_1$-$C_6$ alkyl is $C_1$-$C_3$ alkyl or $C_4$-$C_6$ alkyl. More preferably, $C_1$-$C_3$ alkyl is $C_1$ alkyl ($CH_2$), $C_2$ alkyl (($CH_2$)$_2$), $C_3$ alkyl (e.g. $(CH_2)_3$), and $C_4$-$C_6$ alkyl is $C_4$ alkyl (e.g. $(CH_2)_4$), $C_5$ alkyl (e.g. $(CH_2)_5$) or $C_6$ alkyl (e.g. $(CH_2)_6$).

The alkyl chain A can be substituted at any position with E. Preferably, the alkyl chain is substituted with E at the terminal position of the chain.

Preferably, E is CO-NH or NH-CO,
More preferably, E is CO-NH. Thus, more preferably, F-aryl-E-A is F-aryl-CO-NH-A or F-heteroaryl-E-A is F-heteroaryl-CO-NH-A.

Preferably, $C_6$-$C_{10}$ mono- or bicyclic aryl is F-$C_6H_4$- or F-$C_{10}H_6$-(1-naphthyl- or 2-naphthyl-) optionally substituted by 1 or 2 substituents independently selected from halogen, $NO_2$, CN, COOH, ($C_1$-$C_3$)alkyl, formyl, acetyl, alkoxycarbonyl or trifluoromethyl. Aryl can be substituted at any position with the Fluorine atom (F).

Preferably, F-mono- or bicyclic heteroaryl is F-pyridinyl- or F-pyrimidinyl-. Heteroaryl can be substituted at any carbon atom with the Fluorine atom (F).

Embodiments and preferred features can be combined together and are within the scope of the invention.

Invention compounds are selected from but not limited to

(2*S*,4*S*)-2-Amino-4-{3-[(2-fluoro-pyridine-4-carbonyl)-amino]-propyl}-pentanedioic acid

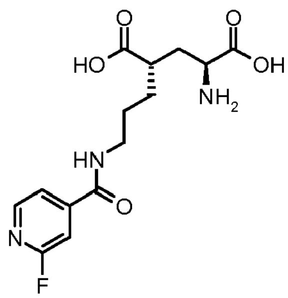

(2*S*,4*S*)-2-Amino-4-{3-[(2-[18F]fluoro-pyridine-4-carbonyl)-amino]-propyl}-pentanedioic acid

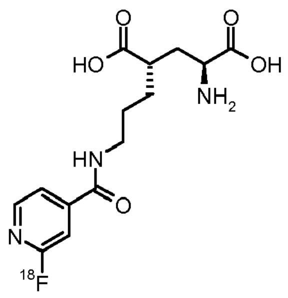

(2*S*,4*S*)-2-Amino-4-{3-[(6-fluoro-pyridine-3-carbonyl)-amino]-propyl}-pentanedioic acid

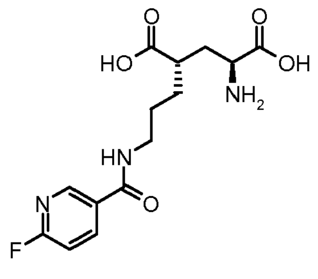

(2*S*,4*S*)-2-Amino-4-{3-[(6-[18F]fluoro-pyridine-3-carbonyl)-amino]-propyl}-pentanedioic acid

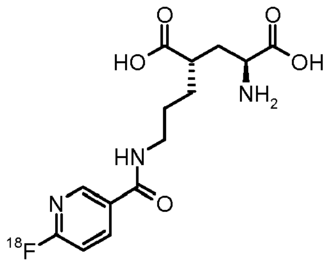

(2*S*,4*S*)-2-Amino-4-[3-(3-cyano-4-fluoro-benzoylamino)-propyl]-pentanedioic acid

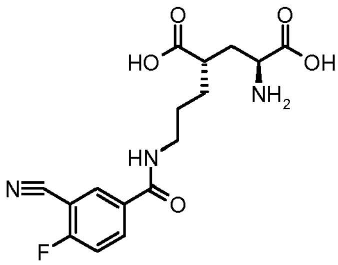

(2*S*,4*S*)-2-Amino-4-[3-(3-cyano-4-[18F]fluoro-benzoylamino)-propyl]-pentanedioic acid

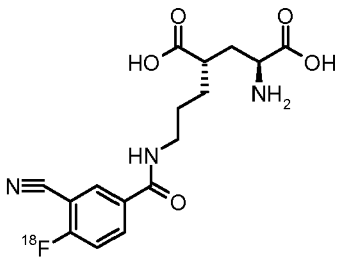

In a **second** aspect, the invention is directed to compounds of the general formula (II)

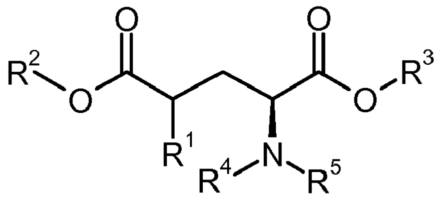

(II)

wherein $R^1$ is
F-aryl-E-A
wherein E is CO, CO-NH or NH-CO
and A is $C_1$-$C_6$ alkyl wherein the carbon chain may be interrupted by one oxygen atom with the proviso that there are at least two carbon atoms between two hetero atoms and
wherein the aryl moiety is a mono- or bicyclic aryl optionally substituted by 1 or 2 substituents independently selected from halogen, $NO_2$, CN, COOH, $(C_1$-$C_3)$alkyl, formyl, acetyl, alkoxycarbonyl or trifluoromethyl;
or
F-heteroaryl-E-A

wherein the heteroaryl moiety is a mono- or bicyclic heteroaryl wherein heteroaryl comprises 5 to 10 ring atoms wherein 1 or 2 ring atoms are independently selected from N, O or S and wherein the heteroaryl moiety is optionally substituted by 1 or 2 methyl groups;

$R^2$ = Hydrogen or O-protecting group;

$R^3$ = Hydrogen or O-protecting group;

$R^4$ = Hydrogen or N-protecting group;

$R^5$ = Hydrogen or N-protecting group;

or the group $NR^4R^5$ is a 1,3-dioxo-1,3-dihydro-2H-isoindol-2-yl (phthalimido) or an azido group;

with the proviso, that at least one of the substituents $R^2$, $R^3$, $R^4$, or $R^5$ is not Hydrogen.

Formula II encompasses single isomers, diastereomers, enantiomers, mixtures thereof and suitable salts thereof.

In a **first** embodiment, the invention is directed to a compound of general formula (II) wherein the Fluorine atom (F) is $^{18}F$ isotope.

In a **second** embodiment, the invention is directed to a compound of general formula (II) wherein the Fluorine atom (F) is $^{19}F$ isotope.

The compounds of formula II are fluorine labeled compounds wherein the functional group(s) such as OH and $NH_2$ are protected with suitable protecting group(s) defined as $R^2$ to $R^5$, respectively.

O-protecting groups selected from the group comprising

Methyl, Ethyl, Propyl, Butyl, t-Butyl, Allyl, Benzyl, 4-Methoxybenzyl, and 4-Methoxyphenyl. Preferably, O-protecting groups are selected from the group comprising Methyl, Ethyl, t-Butyl, and Benzyl. Preferably, $R^2$ and $R^3$ are O-protecting groups. More preferably, $R^2$ and $R^3$ are t-Butyl.

N-protecting groups are selected from the group comprising

Carbobenzyloxy (Cbz), p-Methoxybenzyl carbonyl (Moz or MeOZ), tert-Butyloxycarbonyl (BOC), 9-Fluorenylmethyloxycarbonyl (FMOC), Benzyl (Bn), p-Methoxybenzyl (PMB), 3,4-Dimethoxybenzyl (DMPM), Triphenylmethyl and p-methoxyphenyl (PMP) or the group $NR^4R^5$ is a 1,3-dioxo-1,3-dihydro-2H-isoindol-2-yl (phthalimido) or an azido group.

Preferably, $R^4$ and $R^5$ are selected independently from each other from the group comprising Carbobenzyloxy (Cbz), p-Methoxybenzyl carbonyl (Moz or MeOZ), tert-Butyloxycarbonyl (BOC), 9-Fluorenylmethyloxycarbonyl (FMOC), Benzyl (Bn).

More preferably, $R^4$ and $R^5$ are selected independently from each other from the group comprising tert-Butyloxycarbonyl (BOC) and 9-Fluorenylmethyloxycarbonyl (FMOC).

Even more preferably, $R^4$ is an N-protecting group and $R^5$ is Hydrogen or an N-protecting group.

The preferred feature disclosed for $R^1$ in compounds of general formula (I) is incorporated herein.

Invention compounds are selected from but not limited to

(2*S*,4*S*)-2-tert-Butoxycarbonylamino-4-{3-[(2-[18F]fluoro-pyridine-4-carbonyl)-amino]-propyl}-pentanedioic acid di-*tert*-butyl ester

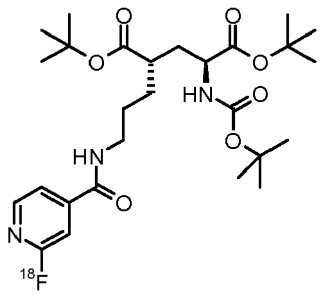

(2*S*,4*S*)-2-tert-Butoxycarbonylamino-4-{3-[(6-[18F]fluoro-pyridine-3-carbonyl)-amino]-propyl}-pentanedioic acid di-*tert*-butyl ester

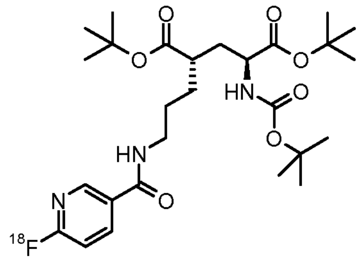

(2*S*,4*S*)-2-tert-Butoxycarbonylamino -4-[3-(3-cyano-4-[18F]fluoro-benzoylamino)-propyl]-pentanedioic acid di-*tert*-butyl ester

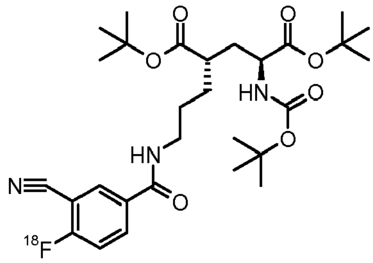

In a **third** aspect, the invention is directed to compounds of the general formula (III)

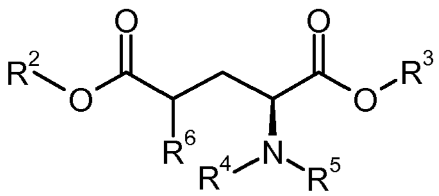

(III)

wherein $R^6$ is
Leaving Group (LG)-aryl-E-A
wherein E is CO, CO-NH or NH-CO
and A is $C_1$-$C_6$ alkyl wherein the carbon chain may be interrupted by one oxygen atom with the proviso that there are at least two carbon atoms between two hetero atoms and
wherein the aryl moiety is a mono- or bicyclic aryl optionally substituted by 1 or 2 substituents independently selected from halogen, $NO_2$, CN, COOH, ($C_1$-$C_3$)alkyl, formyl, acetyl, alkoxycarbonyl or trifluoromethyl;
or
LG-heteroaryl-E-A
wherein the heteroaryl moiety is a mono- or bicyclic heteroaryl wherein heteroaryl comprises 5 to 10 ring atoms wherein 1 or 2 ring atoms are independently selected from N, O or S and wherein the heteroaryl moiety is optionally substituted by 1 or 2 methyl groups;
$R^2$ = Hydrogen or O-protecting group;
$R^3$ = Hydrogen or O-protecting group;
$R^4$ = Hydrogen or N-protecting group;
$R^5$ = Hydrogen or N-protecting group;
or the group $NR^4R^5$ is a 1,3-dioxo-1,3-dihydro-2H-isoindol-2-yl (phthalimido) or an azido group;
with the proviso, that at least one of the substituents $R^2$, $R^3$, $R^4$, or $R^5$ is not Hydrogen.

Formula III encompasses single isomers, diastereomers, enantiomers, mixtures thereof and suitable salts thereof.

The compounds of formula III, are compounds suitable for fluorolabeling wherein the functional group(s) such as OH and $NH_2$ are protected with suitable protecting group(s) such as $R^2$ to $R^5$, respectively.

Preferably, $C_1$-$C_6$ alkyl is $C_1$-$C_3$ alkyl or $C_4$-$C_6$ alkyl. More preferably, $C_1$-$C_3$ alkyl is $C_1$ alkyl ($CH_2$), $C_2$ alkyl (($CH_2$)$_2$), $C_3$ alkyl (e.g. ($CH_2$)$_3$), and $C_4$-$C_6$ alkyl is $C_4$ alkyl (e.g. ($CH_2$)$_4$), $C_5$ alkyl (e.g. ($CH_2$)$_5$) or $C_6$ alkyl (e.g. ($CH_2$)$_6$).

The alkyl chain A can be substituted at any position with E. Preferably, the alkyl chain is substituted with E at the terminal position of the chain.

Preferably, E is CO-NH or NH-CO,
More preferably, E is CO-NH. Thus, more preferably, LG-aryl-E-A is LG-aryl-CO-NH-A or LG-heteroaryl-E-A is LG-heteroaryl-CO-NH-A.

Preferably, $C_6$-$C_{10}$ mono- or bicyclic aryl is LG-$C_6H_4$- or LG-$C_{10}H_6$- (1-naphthyl- or 2-naphthyl-) optionally substituted by 1 or 2 substituents independently selected from halogen, $NO_2$, CN, COOH, ($C_1$-$C_3$)alkyl, formyl, acetyl, alkoxycarbonyl or trifluoromethyl. Aryl can be substituted at any position with the Leaving Group (LG).

Preferably, LG-mono- or bicyclic heteroaryl is LG-pyridinyl- or LG-pyrimidinyl-. Heteroaryl can be substituted at any carbon atom with the Leaving Group (LG).

Embodiments and preferred features can be combined together and are within the scope of the invention.

Preferably, the Leaving Group (LG) is selected from the group of
bromo,
iodo,
nitro,
trimethyl ammonium,
4-methoxyphenyliodonium, and
2-thienyliodonium.

More preferably, the Leaving Group (LG) is selected from the group of
bromo,
iodo, and
trimethyl ammonium.

O-protecting groups selected from the group comprising
Methyl, Ethyl, Propyl, Butyl, t-Butyl, Allyl, Benzyl, 4-Methoxybenzyl, and 4-Methoxyphenyl. Preferably, O-protecting groups are selected from the group comprising Methyl, Ethyl, t-Butyl, and Benzyl. Preferably, $R^2$ and $R^3$ are O-protecting groups. More preferably, $R^2$ and $R^3$ are t-Butyl.

N-protecting groups are selected from the group comprising
Carbobenzyloxy (Cbz), p-Methoxybenzyl carbonyl (Moz or MeOZ), tert-Butyloxycarbonyl (BOC), 9-Fluorenylmethyloxycarbonyl (FMOC), Benzyl (Bn), p-Methoxybenzyl (PMB), 3,4-Dimethoxybenzyl (DMPM), Triphenylmethyl and p-methoxyphenyl (PMP) or the group $NR^4R^5$ is a 1,3-dioxo-1,3-dihydro-2H-isoindol-2-yl (phthalimido) or an azido group.

Preferably, $R^4$ and $R^5$ are selected independently from each other from the group comprising Carbobenzyloxy (Cbz), p-Methoxybenzyl carbonyl (Moz or MeOZ), tert-Butyloxycarbonyl (BOC), 9-Fluorenylmethyloxycarbonyl (FMOC), Benzyl (Bn).

More preferably, $R^4$ and $R^5$ are selected independently from each other from the group comprising tert-Butyloxycarbonyl (BOC) and 9-Fluorenylmethyloxycarbonyl (FMOC).

Even more preferably, $R^4$ is an N-protecting group and $R^5$ is Hydrogen or an N-protecting group.

Invention compounds are selected from but not limited to
(2*S*,4*S*)-2-tert-Butoxycarbonylamino-4-{3-[(2-bromo-pyridine-4-carbonyl)-amino]-propyl}-pentanedioic acid di-*tert*-butyl ester

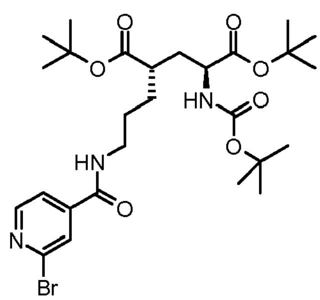

(2*S*,4*S*)-2-tert-Butoxycarbonylamino-4-{3-[(2-iodo-pyridine-4-carbonyl)-amino]-propyl}-pentanedioic acid di-*tert*-butyl ester

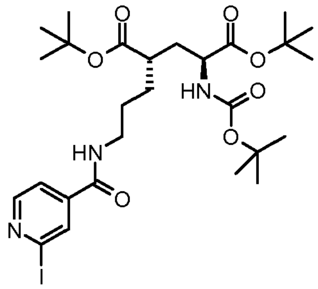

(2*S*,4*S*)-2-tert-Butoxycarbonylamino-4-{3-[(6-nitro-pyridine-3-carbonyl)-amino]-propyl}-pentanedioic acid di-*tert*-butyl ester

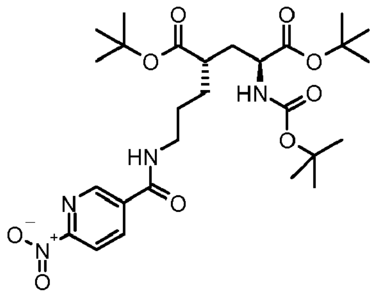

[4-((4*S*,6*S*)-4,6-Bis-tert-butoxycarbonyl-6-tert-butoxycarbonylamino-hexylcarbamoyl)-2-cyano-phenyl]-trimethyl-ammonium; iodide

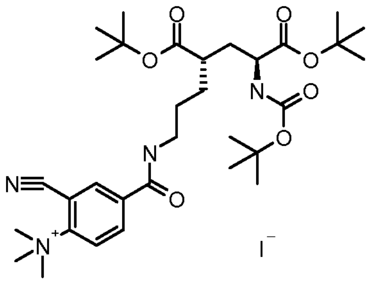

In a **fourth** aspect, the invention is directed to a composition comprising compounds of the general formula (I), (II), (III) or mixtures thereof and pharmaceutically acceptable carrier or diluent.

The person skilled in the art is familiar with auxiliaries, vehicles, excipients, diluents, carriers or adjuvants which are suitable for the desired pharmaceutical formulations, preparations or compositions on account of his/her expert knowledge.

The administration of the compounds, pharmaceutical compositions or combinations according to the invention is performed in any of the generally accepted modes of administration available in the art. Intravenous deliveries are preferred.

Generally, the pharmaceutical compositions according to the invention can be administered such that the dose of the active compound is in the range of 37 MBq (1 mCi) to 740 MBq (20 mCi). In particular, a dose in the range from 150 MBq to 370 MBq will be used.

In a **fifth** aspect, the invention is directed to method for obtaining compound of formula (I), (II) or mixtures thereof.

The method of the invention is a fluoro-labeling method.

Preferably, the fluoro-labeling method concerns a method for labeling invention compounds with Fluorine atom (F) containing moiety wherein the Fluorine atom (F) containing moiety preferably comprises $^{18}F$ or $^{19}F$.

More preferably, Fluorine atom (F) containing moiety comprises $^{18}F$. Even more preferably, the Fluorine atom

(F) containing moiety is 4,7,13,16,21,24-Hexaoxa-1,10-diazabicyclo[8.8.8]-hexacosane K18F (crownether salt Kryptofix K18F), $K^{18}F$, $H^{18}F$, $KH^{18}F_2$, $Cs^{18}F$, $Na^{18}F$ or tetraalkylammonium salt of $^{18}F$ (e.g.[F-18] tetrabutylammonium fluoride). Most preferably, the Fluorine atom (F) containing moiety is $K^{18}F$, $H^{18}F$, or $KH^{18}F_2$.

Preferably, the fluoro-labeling method is a fluoro-radiolabeling method.

Under the present invention, the method is a direct labelling method for obtaining compounds of formula (I), (II) or mixtures thereof.

The fluoro-labeling method comprises the steps

- Coupling a compound of general Formula (III) with a Fluorine containing moiety,
- Deprotecting compound of formula (II) and
- Optionally converting obtained compound into suitable salt of inorganic or organic acids thereof, hydrates and solvates thereof.

Preferably, the method is a direct labelling method for obtaining compounds of formula (I), (II) or mixtures thereof.

The fluoro-labeling method comprises the steps

- Coupling a compound of general Formula (III) with Fluorine containing moiety wherein the Fluorine containing moiety comprises $^{18}F$,
- Deprotecting compound of formula (II) and
- Optionally converting obtained compound into suitable salt of inorganic or organic acids thereof, hydrates and solvates thereof.

Preferably, the method is a direct labelling method for obtaining a compound of formula (I), (II) or mixtures thereof.

The fluoro-labeling method comprises the steps

- Coupling compound of general Formula (III) with Fluorine containing moiety wherein the Fluorine containing moiety comprises $^{19}F$,
- Deprotecting compound of formula (II) and
- Optionally converting obtained compound into suitable salt of inorganic or organic acids thereof, hydrates and solvates thereof.

The reagents, solvents and conditions which can be used for this fluorination are common and well-known to the skilled person in the field. See, *e.g.,* J. Fluorine Chem., 27 (1985):177-191. Preferably, the solvents used in the present method is DMF, DMSO, acetonitrile, DMA, or mixture thereof, preferably the solvent is DMSO.

Compounds of formula (I), (II) and (III) are defined as above wherein embodiment and preferred features are herein enclosed.

In a **sixth** aspect, the invention is directed to compounds of general formula (I) or (II) or mixtures thereof for the manufacture of an imaging tracer for imaging proliferative diseases. In other word, the invention is directed to the use of invention compounds of general formula (I) and (II) for the manufacture of an imaging tracer for imaging proliferative diseases. The compounds of general formula (I) and (II) are herein defined as above and encompass all embodiments and preferred features. Preferably, the invention compounds are compounds of general formula (I) or (II) wherein the Fluorine atom (F) is $^{18}F$ isotope. The imaging tracer is Positron Emission Tomography PET suitable imaging tracer.

The invention is also directed to a method for imaging or diagnosis proliferative diseases comprising the steps:

- Administrating to a mammal an effective amount of a compound comprising compounds of general formula (I) or (II),
- Obtaining images of the mammal and
- Assessing images.

Proliferative diseases are cancer characterised by the presence of tumor and/or metastases. Preferably, tumor are selected from the group of malignomas of the gastrointestinal or colorectal tract, liver carcinoma, pancreas carcinoma, kidney carcinoma, bladder carcinoma, thyroid carcinoma, prostate carcinoma, endometrial carcinoma, ovary carcinoma, testes carcinoma, melanoma, small-cell and non-small-cell bronchial carcinoma, dysplastic oral mucosa carcinoma, invasive oral cancer; breast cancer, including hormone-dependent and hormone-independent breast cancer, squamous cell carcinoma, neurological cancer disorders including neuroblastoma, glioma, astrocytoma, osteosarcoma, meningioma, soft tissue sarcoma; haemangioma and endocrine tumors, including pituitary adenoma, chromocytoma, paraganglioma, haematological tumor disorders including lymphoma and leukaemias; Preferably, the tumor is bronchial carcinoma.

Preferably, metastases are metastases of one of the tumors mentioned above.

Preferably, the invention compounds and use is for manufacturing a PET imaging tracer for imaging tumor in a mammal wherein the tumor is preferably a non-small-cell bronchial carcinoma.

In a **seventh aspect,** the invention is directed to the use of compounds of general formula (I), (II) or (III) for conducting biological assays and chromatographic identification. More preferably, the use relates to compounds of general formula (I) or (II) wherein the fluorine isotope is $^{18}F$ or $^{19}F$, more preferably $^{19}F$.

Compounds of general formula (I), (II) or (III) wherein the fluorine isotope is $^{19}F$ are useful as reference and/or measurement agent.

The compounds of general formula (I), (II) and (III) are herein defined as above and encompass all embodiments and preferred features.

In a **eighth aspect,** the present invention provides a kit comprising a sealed vial containing a predetermined quantity of a compound having general chemical Formula (I), (II) or (III) or mixtures thereof and suitable salts of inorganic or organic acids thereof, hydrates and solvates thereof. Optionally the kit comprises a pharmaceutically acceptable carrier, diluent, excipient or adjuvant.

**Definitions**

The terms used in the present invention are defined below but are not limiting the invention scope.

If chiral centers or other forms of isomeric centers are not otherwise defined in a compound according to the present invention, all forms of such stereoisomers, including enantiomers and diastereoisomers, are intended to be covered herein. Compounds containing chiral centers may be used as racemic mixture or as an enantiomerically enriched mixture or as a diastereomeric mixture or as a diastereomerically enriched mixture, or these isomeric mixtures may be separated using well-known techniques, and an individual stereoisomer maybe used alone.

In the context of the present invention, preferred suitable salts are pharmaceutically acceptable salts of the compounds according to the invention. The invention also comprises salts which for their part are not suitable for pharmaceutical applications, but which can be used, for example, for isolating or purifying the compounds according to the invention. Suitable salts of the compounds according to the invention include acid addition salts of mineral acids, carboxylic acids and sulphonic acids, for example salts of hydrochloric acid, hydrobromic acid, sulphuric acid, phosphoric acid, methanesulphonic acid, ethanesulphonic acid, toluenesulphonic acid, benzenesulphonic acid, naphthalene disulphonic acid, acetic acid, trifluoroacetic acid, propionic acid, lactic acid, tartaric acid, malic acid, citric acid, fumaric acid, maleic acid and benzoic acid.

Suitable salts of the compounds according to the invention also include salts of customary bases, such as, by way of example and by way of preference, alkali metal salts (for example sodium salts and potassium salts), alkaline earth metal salts (for example calcium salts and magnesium salts) and ammonium salts, derived from ammonia or organic amines having 1 to 16 carbon atoms, such as, by way of example and by way of preference, ethylamine, diethylamine, triethylamine, ethyldiisopropylamine, monoethanolamine, dietha-nolamine, triethanolamine, dicyclohexylamine, dimethylaminoethanol, procaine, diben-zylamine, N methylmorpholine, arginine, lysine, ethylenediamine and N methylpiperidine.

The term "$C_1$-$C_6$ alkyl", used herein on its own or as part of another group, refers to saturated carbon chains which may be straight-chain or branched, in particular to methyl, ethyl, n-propyl, isopropyl, n-butyl, isobutyl, tert-butyl, methylpropyl, n-pentyl, 2,2-dimethylpropyl, 2-methylbutyl, 3-methylbutyl or n-hexyl.

The term "aryl" as employed herein by itself or as part of another group refers to monocyclic or bicyclic $C_6$-$C_{10}$ aromatic rings, in particular phenyl or naphthyl groups e.g. 1-naphthyl and 2-naphthyl, which themselves can be substituted with one or two substituents independently and individually selected from but not limited to the group comprising halogen, $NO_2$, CN, COOH, ($C_1$-$C_3$)alkyl, formyl, acetyl, alkoxycarbonyl or trifluoromethyl.

The term "heteroaryl" as employed herein by itself or as part of another group refers to monocyclic or bicyclic heteroaromatic groups containing from 5 to 10 ring atoms, wherein 1 or 2 atoms of the ring portion are independently selected from N, O or S, e.g. thienyl, furanyl, pyrrolyl, oxazolyl, thiazolyl, imidazolyl, pyrazolyl, isoxazolyl, isothiazolyl, benzofuranyl, benzothienyl, benzothiazolyl, benzoxazolyl, benzimidazolyl, indazolyl, indolyl, pyridyl, pyridazinyl, pyrimidinyl, pyrazinyl, quinolyl, isoquinolyl etc.; which themselves can be substituted with one or two methyl groups.

Halogen as used herein refers to fluoro, chloro, bromo or iodo.

The term "amine-protecting group" as employed herein by itself or as part of another group is known or obvious to someone skilled in the art, which is chosen from but not limited to a class of protecting groups namely carbamates, amides, imides, N-alkyl amines, N-aryl amines, imines, enamines, boranes, N-P protecting groups, N-sulfenyl, N-sulfonyl and N-silyl, and which is chosen from but not limited to those described in the textbook Greene and Wuts, Protecting groups in Organic Synthesis, third edition, page 494-653, included herewith by reference.

Amino protecting groups are selected from the group comprising

Carbobenzyloxy (Cbz), *p*-Methoxybenzyl carbonyl (Moz or MeOZ), *tert*-Butyloxycarbonyl (BOC), 9-Fluorenylmethyloxycarbonyl (FMOC), Benzyl (Bn), *p*-Methoxybenzyl (PMB), 3,4-Dimethoxybenzyl (DMPM), *p*-methoxyphenyl (PMP) or the

protected amino group is a 1,3-dioxo-1,3-dihydro-2H-isoindol-2-yl (phthalimido) or an azido group.

O-protecting groups are selected from the group comprising Methyl, Ethyl, Propyl, Butyl, t-Butyl, Allyl, Benzyl, 4-Methoxybenzyl, 4-Methoxyphenyl.

General synthesis of F-18 compounds

The radiofluorination reaction can be carried out, for example in a typical reaction vessel (e.g. Wheaton vial) which is known to someone skilled in the art or in a microreactor. The reaction can be heated by typical methods, e.g. oil bath, heating block or microwave. The radiofluorination reactions are carried out in dimethylformamide with potassium carbonate as base and "kryptofix" as crown-ether. But also other solvents can be used which are well known to experts. These possible conditions include, but are not limited to: dimethylsulfoxide and acetonitrile as solvent and tetraalkyl ammonium and tertraalkyl phosphonium carbonate as base. Water and/or alcohol can be involved in such a reaction as co-solvent. The radiofluorination reactions are conducted for one to 60 minutes. Preferred reaction times are five to 50 minutes. Further preferred reaction times are 10 to 40 min. This and other conditions for such radiofluorination are known to experts (Coenen, Fluorine-18 Labeling Methods: Features and Possibilities of Basic Reactions, (2006), in: Schubiger P.A., Friebe M., Lehmann L., (eds), PET-Chemistry - The Driving Force in Molecular Imaging. Springer, Berlin Heidelberg, pp.15-50). The radiofluorination can be carried out in a "hot-cell" and/or by use of a module (eview: Krasikowa, Synthesis Modules and Automation in F-18 labeling (2006), in: Schubiger P.A., Friebe M., Lehmann L., (eds), PET-Chemistry - The Driving Force in Molecular Imaging. Springer, Berlin Heidelberg, pp. 289-316) which allows an automated or semi-automated synthesis.

Precursors for F-18-aryl-or F-18-heteroaryl-compounds of general formula I are e.g. aryl or heteroaryl bromides, iodides, nitro compounds, trialkyl ammonium, aryliodonium which can be converted to the respective F-18 compounds of this invention by methods known in the art (L. Cai, S. Lu, V. Pike, Eur. J. Org. Chem 2008, 2853-2873). Starting materials for these precursors are commercially available or can be synthesized by methods known in the art (R.C. Larock, Comprehensive Organic Transformations, VCH Publishers 1989).

The $^{19}$F-compounds can be synthesized according to the syntheses for the $^{18}$F-compounds (see above), by deoxofluorination reactions (M. Hudlicky, Org. React. 1988, 35, 513; H. Vorbrüggen, Synthesis 2008, 8, 1165-1174.), by electrophilic fluorinations (T. Suzuki et al., J. Org. Chem. 2007, 72, 246-250.), or by employment of fluorine-bearing building block.

## Experimental Section

### Abbreviations

| | |
|---|---|
| Br | broad signal (in NMR) |
| D | doublet |
| Dd | doublet of doublet |
| DMA | *N,N*-dimethylacetamide |
| DMF | *N,N*-dimethylformamide |
| DMSO | dimethylsulphoxide |
| Dt | doublet of triplet |
| ESI | Electrospray ionisation |
| MS | Mass spectrometry |
| M | multiplet |
| MeCN | acetonitrile |
| NMR | Nuclear magnetic resonance spectroscopy: chemical shifts (δ) are given in ppm. |
| r.t. | room temperature |
| S | Singlet |
| T | Triplet |

(continued)

| | |
|---|---|
| THF | Tetrahydrofurane |
| TFA | Trifluoro acetic acid |

**Examples:**

**Example 1**

**(2*S*,4*S*)-2-Amino-4-{3-[(2-fluoro-pyridine-4-carbonyl)-amino]-propyl}-pentanedioic acid**

a) Di-*tert*-butyl (2*S*,4*S*)-4-allyl-2-tert-butoxycarbonylamino-pentanedioate

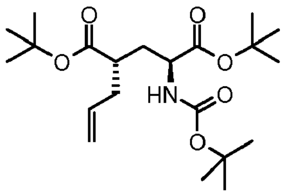

26.96 g (75 mmol) of di-*tert*-butyl Boc-glutamate (Journal of Peptide Research (2001), 58, 338) were dissolved in 220 mL of tetrahydrofuran (THF) and cooled to -70°C. 165 mL (165 mmol) of a 1M solution of lithium bis(trimethylsilyl)amide in THF were added dropwise over a period of two hours at this temperature and the mixture was stirred at -70°C for another 2 hours. 27.22 g (225 mmol) of allyl bromide were then added dropwise, and after 2 h at this temperature, the cooling bath was removed and 375 mL of 2N aqueous hydrochloric acid and 1.25 L of ethyl acetate were added. The organic phase was separated off, washed with water until neutral, dried over sodium sulphate and filtered, and the filtrate was concentrated. The crude product obtained in this manner was chromatographed in silica gel using a hexane/ethyl acetate gradient, and the appropriate fractions were combined and concentrated.
Yield: 15.9 g (53.1%)
MS (ESIpos): m/z = 400 $[M+H]^+$
1H NMR (300 MHz, CHLOROFORM-d) d ppm 1.32-1.58 (m, 27H) 1.81-1.92 (m, 2H) 2.25-2.39 (m, 2H) 2.40-2.48 (m, 1H), 4.10-4.18 (m, 1 H) 4.85-4.92 (d, 1H) 5.02-5.11 (m, 2H) 5.68-5.77 (m, 1H)

b) Di-*tert*-butyl (2*S*,4*S*)-2-tert-butoxycarbonylamino-4-(3-hydroxypropyl)-pentanedioate

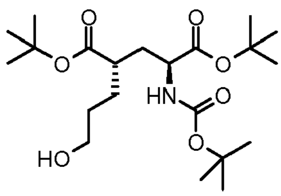

15,58 g (39 mmol) of di-*tert*-butyl (2*S*,4*S*)-4-allyl-2-tert-butoxycarbonylamino-pentanedioate were dissolved in 200 mL of tetrahydrofuran and cooled in an ice-bath. Over a period of about 20 minutes, 54.6 mL (54.6 mmol) of 1 M diboran/tetrahydrofuran complex in tetrahydrofuran were added dropwise with ice-cooling and under nitrogen, and the mixture was stirred on ice for 2 h and at room temperature overnight. It was cooled again to 0°C and 58.5 mL of 1 N aqueous sodium hydroxide solution and 58.5 mL of 30% aqueous hydrogen peroxide solution were then added dropwise. After

30 minutes, the mixture was diluted with water, the tetrahydrofuran was distilled off and the remaining aqueous solution was extracted with ethyl acetate. The organic phase was separated off, washed with water until neutral, dried over sodium sulphate and filtered, and the filtrate was concentrated. The crude product obtained in this manner was chromatographed on silica gel using a hexane/ethyl acetate gradient, and the appropriate fractions were combined and concentrated.

Yield: 8,5 g (52,2 %)
MS (ESIpos): m/z = 418 $[M+H]^+$
1H NMR (300 MHz, CHLOROFORM-d) d ppm 1.32-1.58 (m, 27H) 1.60-1.70 (m, 2H) 1.73-1.94 (m, 4H) 2.05-2.12 (m, 1H), 2.33-2.40 (m, 1H) 3.58-3.68 (m, 2H) 4.15-4.22 (m, 1H)4.95-5.03 (d, 1H)

c) Di-*tert*-butyl (2*S*,4*S*)-2-tert-butoxycarbonylamino-4-(3-methylsulfonyloxy-propyl)-pentanedioate

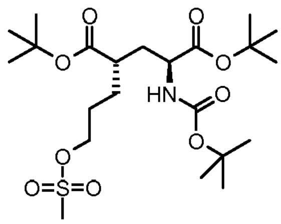

418 mg (1 mmol) of di-*tert*-butyl (2*S*,4*S*)-2-tert-butoxycarbonylamino-4-(3-hydroxypropyl)-pentanedioate was dissolved in 20 mL of dichloromethane and cooled in an ice-bath. After addition of 0.83 mL (6 mmol) of triethylamine and 229 mg (2 mmol) of methanesulphonyl chloride, the mixture was stirred on ice for 2 h and then concentrated. The crude product obtained in this manner was chromatographed on silica gel using a hexane/ethyl acetate gradient and the appropriate fractions were combined and concentrated.
Yield: 350 mg (70.6%)
MS (ESIpos): m/z = 496 $[M+H]^+$
1H-NMR (300MHz, CHLOROFORM-d): Shift [ppm]= 1.44-147 (m, 27H), 1.61-1.96 (m, 6H), 2.32-2.41 (m, 1H), 3.02 (s, 3H), 4.11-4.18 (m, 2H), 4.88-4.91 (d, 1H).

d) Di-*tert*-butyl (2*S*,4*S*)-2-tert-butoxycarbonylamino-4-(3-aminopropyl)-pentanedioate

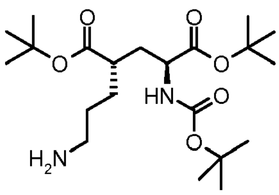

0.90 g (1.8 mmol) of di-*tert*-butyl (2*S*,4*S*)-2-tert-butoxycarbonylamino-4-(3-hydroxypropyl)-pentanedioate **(1c)** and 0.36 g (5.5 mmol) of sodium azide were dissolved in 15 mL of DMF and stirred overnight at 60°C and then concentrated. The residue was taken up in ethyl acetate and water, the organic phase was separated off, washed with water until neutral, dried over sodium sulphate and filtered, and the filtrate was concentrated. The azide obtained (0.73 g) was dissolved in 18 mL of dioxane/methanol (5:1) and 0.78 g (3 mmol) of triphenyl phosphine were added and the mixture was stirred for one hour at room temperature and another 18 h after addition of 1.5 mL (19.5 mmol) concentrated aqueous ammonia solution. The reaction mixture was concentrated *in vacuo* and the residue was taken up in ethyl acetate and water, the organic phase was separated off, washed with water until neutral, dried over sodium sulphate and filtered,

and the filtrate was concentrated. The crude product obtained in this manner was chromatographed on silica gel using a dichloromethane/methanol gradient and the appropriate fractions were combined and concentrated. Yield: 0.45 g (65%)
MS (ESIpos): m/z = 417 $[M+H]^+$
1H-NMR (300MHz, CHLOROFORM-d): Shift [ppm]= 1.44-1.67 (m, 33H), 1.83-1.90 (m, 2H), 2.29-2.39 (m, 1H), 2.71-2.76 (s, 2H), 4.10-4.18 (m, 1H), 4.91-4.94 (m, 1H)

e) (2*S*,4*S*)-2-tert-Butoxycarbonylamino-4-{3-[(2-fluoro-pyridine-4-carbonyl)-amino]-propyl}-pentanedioic acid di-tert-butyl ester

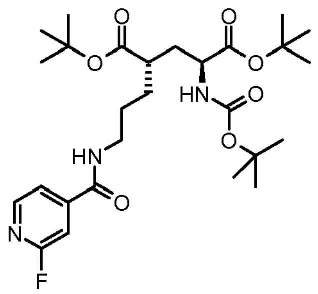

To a solution of 169 mg (1.2 mmol) of 2-fluoro-4-pyridinecarboxylic acid (Aldrich) and 480 mg (1.15 mmol) of di-*tert*-butyl (2*S*,4*S*)-2-tert-butoxycarbonylamino-4-(3-aminopropyl)-pentane-dioate **(1d)** in 30 mL DMF are added 624 mg (1.2 mmol) of (Benzotriazol-1-yloxy)-tripyrrolidinophosphonium hexafluorophosphate (PyBOP) and 0.85 mL N-ethyl-N,N-diisopropylamine and the reaction mixture was stirred overnight at room temperature. After evaporation of the solvent the residue was chromatographed on silica gel using an ethyl acetate/ethanol gradient.
Yield: 518 mg (83.3 %).
MS (ESIpos): m/z = 540 $[M+H]^+$
1H-NMR (400MHz, DMSO-d6): Shift [ppm]= 1.29-1.44 (m, 27H), 1.45-1.56 (m, 4H), 1.62-1.83 (m, 2H), 2.21-2.35 (m, 1H), 3.20-3.30 (s, 4H), 3.71-3.88 (m, 1H), 7.03-7.08 (m, 1H), 7.47-7.50 (m, 1H), 7.68-7.70 (m, 1H), 8.36-8.40 (m, 1H), 8.80-8.88 (m, 1H).

f) (2*S*,4*S*)-2-Amino-4-{3-[(2-fluoro-pyridine-4-carbonyl)-amino]-propyl}-pentanedioic acid

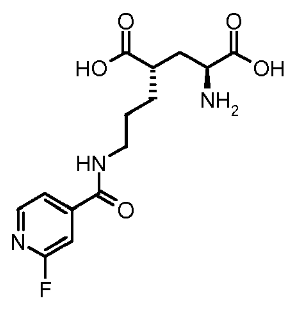

120 mg (0.22 mmol) of (2*S*,4*S*)-2-tert-Butoxycarbonylamino-4-{3-[(2-fluoro-pyridine-4-carbonyl)-amino]-propyl}-pentanedioic acid di-tert-butyl ester **(1e)** were treated with 12 mL (24 mmol) of 2M hydrochloric acid and stirred for 3d at room temperature. The precipitate was collected by filtration and the colorless solid was washed with ether and dried *in vacuo.*
The crude product obtained in this manner was chromatographed on C18 silica gel using a water/methanol

gradient, and the appropriate fractions were combined and concentrated. Yield: 11 mg (15.1 %).

MS (ESIpos): m/z = 328 $[M+H]^+$

**Example 2: Cell-uptake experiments**

The ability of compounds from the present invention to compete with uptake of glutamic acid into tumor cells was examined. Therefore, tumor cells were co-incubated with 3H-labeled glutamic acid and (2*S*,4*S*)-2-Amino-4-{3-[(2-fluoro-pyridine-4-carbonyl)-amino]-propyl}-pentanedioic acid. This compound was used in excess (1mM) to the tracer 3H-glutamic acid. Interestingly, (2*S*,4*S*)-2-Amino-4-{3-[(2-fluoro-pyridine-4-carbonyl)-amino]-propyl}-pentane-dioic acid was able to reduce the uptake of glutamic acid to a large extent, indicating that the same transport systems may be exploited by the test-compound. See figure 1.

## Claims

1. A compound of the general formula (I)

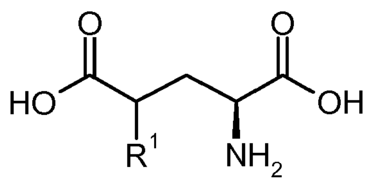

(I)

wherein $R^1$ is
F-aryl-E-A
wherein E is CO, CO-NH or NH-CO
and A is $C_1$-$C_6$ alkyl wherein the carbon chain may be interrupted by one oxygen atom with the proviso that there are at least two carbon atoms between two hetero atoms and
wherein the aryl moiety is a mono- or bicyclic aryl optionally substituted by 1 or 2 substituents independently selected from halogen, $NO_2$, CN, COOH, ($C_1$-$C_3$)alkyl, formyl, acetyl, alkoxycarbonyl or trifluoromethyl;
or
F-heteroaryl-E-A
wherein the heteroaryl moiety is a mono- or bicyclic heteroaryl wherein heteroaryl comprises 5 to 10 ring atoms wherein 1 or 2 ring atoms are independently selected from N, O or S and wherein the heteroaryl moiety is optionally substituted by 1 or 2 methyl groups;
and encompassing single isomers, diastereomers and enantiomers, mixtures thereof and suitable salts thereof.

2. The compound according to claim 1 selected from (2*S*,4*S*)-2-Amino-4-{3-[(2-fluoro-pyridine-4-carbonyl)-amino]-propyl}-pentanedioic acid

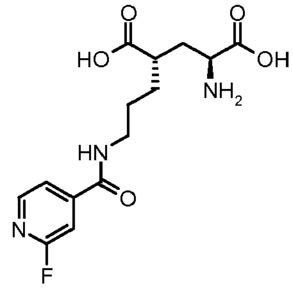

(2*S*,4*S*)-2-Amino-4-{3-[(2-[18F]fluoro-pyridine-4-carbonyl)-amino]-propyl}-pentanedioic acid

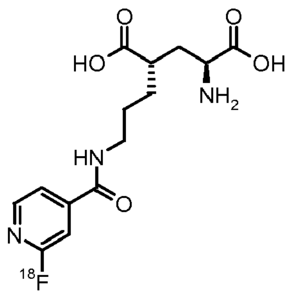

(2*S*,4*S*)-2-Amino-4-{3-[(6-fluoro-pyridine-3-carbonyl)-amino]-propyl}-pentanedioic acid

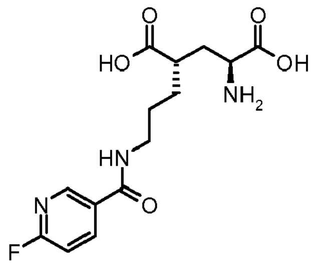

(2*S*,4*S*)-2-Amino-4-{3-[(6-[18F]fluoro-pyridine-3-carbonyl)-amino]-propyl}-pentanedioic acid

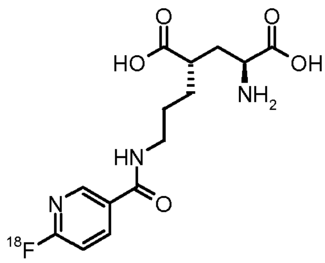

(2*S*,4*S*)-2-Amino-4-[3-(3-cyano-4-fluoro-benzoylamino)-propyl]-pentanedioic acid

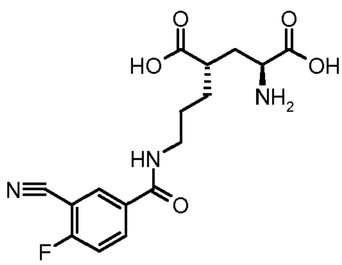

and

(2*S*,4*S*)-2-Amino-4-[3-(3-cyano-4-[18F]fluoro-benzoylamino)-propyl]-pentanedioic acid

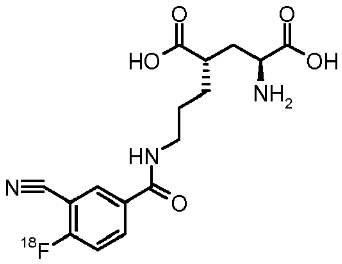

3. A compound of the general formula (II)

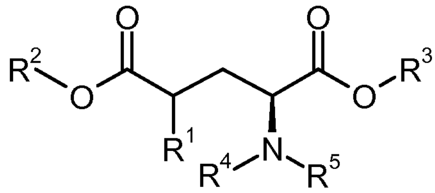

(II)

wherein $R^1$ is
F-aryl-E-A
wherein E is CO, CO-NH or NH-CO
and A is $C_1$-$C_6$ alkyl wherein the carbon chain may be interrupted by one oxygen atom with the proviso that there are at least two carbon atoms between two hetero atoms and
wherein the aryl moiety is a mono- or bicyclic aryl optionally substituted by 1 or 2 substituents independently selected from halogen, $NO_2$, CN, COOH, ($C_1$-$C_3$)alkyl, formyl, acetyl, alkoxycarbonyl or trifluoromethyl;
or
F-heteroaryl-E-A
wherein the heteroaryl moiety is a mono- or bicyclic heteroaryl wherein heteroaryl comprises 5 to 10 ring atoms wherein 1 or 2 ring atoms are independently selected from N, O or S and wherein the heteroaryl moiety is optionally substituted by 1 or 2 methyl groups;
$R^2$ = Hydrogen or O-protecting group;
$R^3$ = Hydrogen or O-protecting group;
$R^4$ = Hydrogen or N-protecting group;
$R^5$ = Hydrogen or N-protecting group;
or the group $NR^4R^5$ is a 1,3-dioxo-1,3-dihydro-2H-isoindol-2-yl (phthalimido) or an azido group;
with the proviso, that at least one of the substituents $R^2$, $R^3$, $R^4$, or $R^5$ is not Hydrogen;
and encompassing single isomers, diastereomers, enantiomers, mixtures thereof and suitable salts thereof.

4. The compound according to claim 3 selected from
(2*S*,4*S*)-2-tert-Butoxycarbonylamino-4-{3-[(2-[18F]fluoro-pyridine-4-carbonyl)-amino]-propyl}-pentanedioic acid di-*tert*-butyl ester

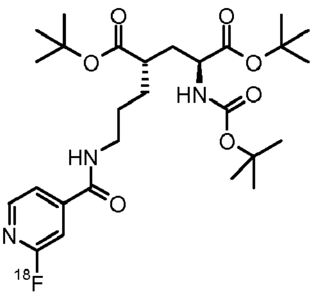

(2*S*,4*S*)-2-tert-Butoxycarbonylamino-4-{3-[(6-[18F]fluoro-pyridine-3-carbonyl)-amino]-propyl}-pentanedioic acid

di-*tert*-butyl ester

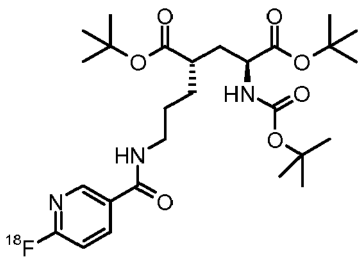

and
(2*S*,4*S*)-2-tert-Butoxycarbonylamino-4-[3-(3-cyano-4-[18F]fluoro-benzoylamino)-propyl]-pentanedioic acid di-*tert*-butyl ester

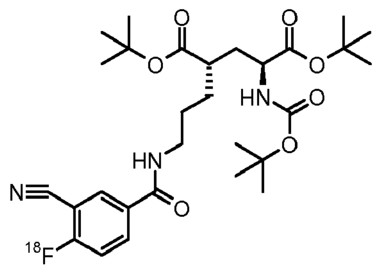

**5.** A compound of the general formula (III)

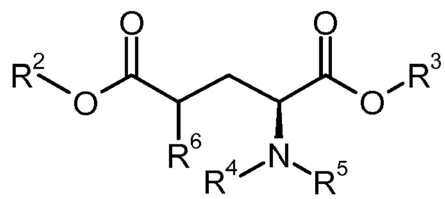

(III)

wherein $R^6$ is
Leaving Group (LG)-aryl-E-A
wherein E is CO, CO-NH or NH-CO
and A is $C_1$-$C_6$ alkyl wherein the carbon chain may be interrupted by one oxygen atom with the proviso that there are at least two carbon atoms between two hetero atoms and
wherein the aryl moiety is a mono- or bicyclic aryl optionally substituted by 1 or 2 substituents independently selected from halogen, $NO_2$, CN, COOH, ($C_1$-$C_3$)alkyl, formyl, acetyl, alkoxycarbonyl or trifluoromethyl;
or
Leaving Group (LG)-heteroaryl-E-A
wherein the heteroaryl moiety is a mono- or bicyclic heteroaryl wherein heteroaryl comprises 5 to 10 ring atoms wherein 1 or 2 ring atoms are independently selected from N, O or S and wherein the heteroaryl moiety is optionally substituted by 1 or 2 methyl groups;
$R^2$ = Hydrogen or O-protecting group;
$R^3$ = Hydrogen or O-protecting group;
$R^4$ = Hydrogen or N-protecting group;
$R^5$ = Hydrogen or N-protecting group;
or the group $NR^4R^5$ is a 1,3-dioxo-1,3-dihydro-2H-isoindol-2-yl (phthalimido) or an azido group;
with the proviso, that at least one of the substituents $R^2$, $R^3$, $R^4$, or $R^5$ is not Hydrogen; and

encompassing single isomers, diastereomers, enantiomers, mixtures thereof and suitable salts thereof.

**6.** The compound according to claim 5 selected from (2*S*,4*S*)-2-tert-Butoxycarbonylamino-4-{3-[(2-bromo-pyridine-4-carbonyl)-amino]-propyl}-pentanedioic acid di-*tert*-butyl ester

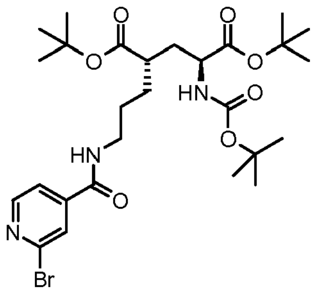

(2*S*,4*S*)-2-tert-Butoxycarbonylamino-4-{3-[(2-iodo-pyridine-4-carbonyl)-amino]-propyl}-pentanedioic acid di-*tert*-butyl ester

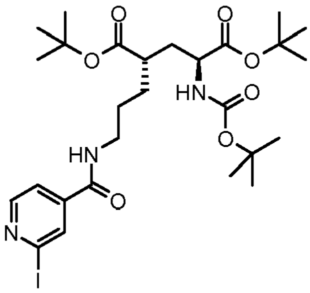

(2*S*,4*S*)-2-tert-Butoxycarbonylamino-4-{3-[(6-nitro-pyridine-3-carbonyl)-amino]-propyl}-pentanedioic acid di-*tert*-butyl ester

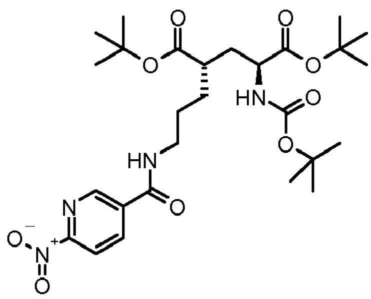

and

[4-((4*S*,6*S*)-4,6-Bis-tert-butoxycarbonyl-6-tert-butoxycarbonylamino-hexylcarbamoyl)-2-cyano-phenyl]-trimethyl-ammonium; iodide

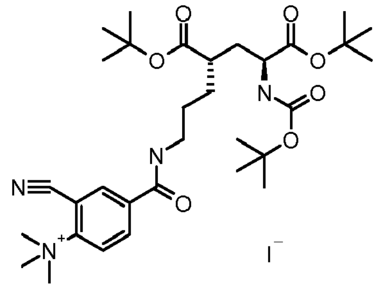

**7.** A composition comprising compounds of the general formula (I), (II), (III) or mixtures thereof according to claims 1, 3 and 5 and pharmaceutically acceptable carrier or diluent.

**8.** A method for obtaining compounds of formula (I), (II) or mixtures thereof according to claims 1 and 3 comprising the steps

- Coupling a compound of general Formula (III) according to claim 5 with a Fluorine containing moiety,
- Optionally deprotecting compound of formula (II) and
- Optionally converting obtained compound into suitable salt of inorganic or organic acids thereof, hydrates and solvates thereof.

**9.** A compound of general formula (I) or (II) or mixtures thereof according to claims 1 and 3 for the manufacture of an imaging tracer for imaging proliferative diseases.

**10.** Use of compounds of general formula (I), (II) or (III) or mixtures thereof according to claims 1, 3 and 5 for conducting biological assays and chromatographic identification.

**11.** A kit comprising a sealed vial containing a predetermined quantity of a compound having general chemical Formula (I), (II) or (III) or mixtures thereof according to claims 1, 3 and 5 and suitable salts of inorganic or organic acids thereof, hydrates and solvates thereof.

**Figure 1:**

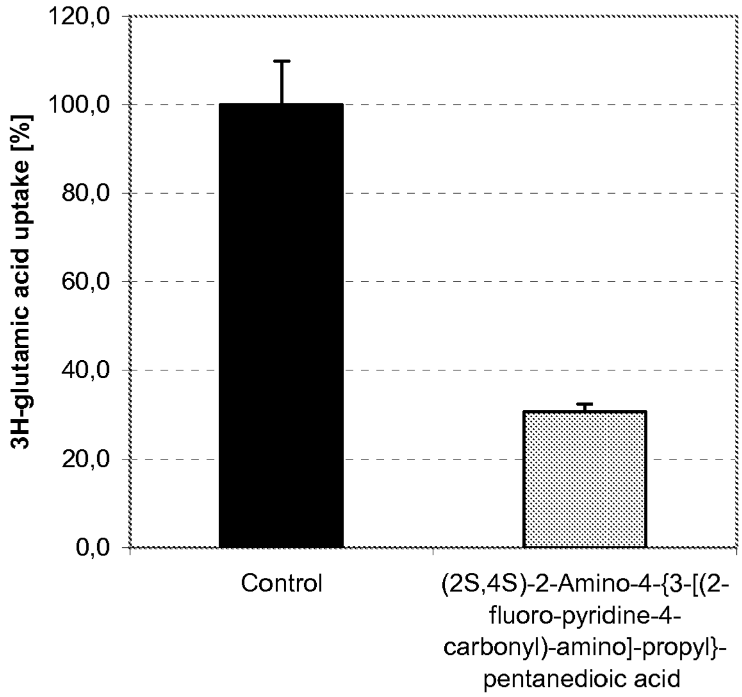
120,0
100,0
80,0
60,0
40,0
20,0
0,0
3H-glutamic acid uptake [%]
Control
(2S,4S)-2-Amino-4-{3-[(2-fluoro-pyridine-4-carbonyl)-amino]-propyl}-pentanedioic acid

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **Warburg O.** Über den Stoffwechsel der Carcinomzelle. *Biochem.Zeitschrift,* 1924, vol. 152, 309-339
- **Kellof G.** Progress and Promise of FDG-PET Imaging for Cancer Patient Management and Oncologic Drug Development. *Clin. Cancer Res.,* 2005, vol. 11 (8), 2785-2807
- *Eur. J. Nucl. Med. Mol. Imaging,* May 2002, vol. 29 (5), 681-90
- *J. Nucl. Med.,* 1991, vol. 32, 1338-1346
- *J. Nucl. Med.,* 1996, vol. 37, 320-325
- *J. Nucl. Med.,* 2001, vol. 42, 752-754
- *J. Nucl. Med.,* 1999, vol. 40, 331-338
- *J. Nucl. Med. Technol.,* 2005, vol. 33, 145-155
- **Gambhir et al.** A tabulated summary of the FDG PET literature. *J. Nucl. Med.,* 2001, vol. 42, 1-93
- *Eur. J. Nucl. Med. Mol. Imaging.,* May 2002, vol. 29 (5), 681-90
- **Medina.** *J. Nutr.,* 2001, vol. 1131, 2539S-2542S
- **Souba.** *Ann Surg,* 1993, vol. 218, 715-728
- **Antoni.** Enzyme Catalyzed Synthesis of L-[4-C-11]aspartate and L-[5-C-11]glutamate. *J. Labeled Compd. Radiopharm.,* 2001, vol. 44 (4), 287-294
- **Buchanan.** The biosynthesis of showdomycin: studies with stable isotopes and the determination of principal precursors. *J. Chem. Soc. Chem. Commun.,* 1984, vol. 22, 1515-1517
- *J. Fluorine Chem.,* 1985, vol. 27, 177-191
- **Greene ; Wuts.** Protecting groups in Organic Synthesis. 494-653
- Fluorine-18 Labeling Methods: Features and Possibilities of Basic Reactions. **Coenen.** PET-Chemistry - The Driving Force in Molecular Imaging. Springer, 2006, 15-50
- Synthesis Modules and Automation in F-18 labeling. **Krasikowa.** PET-Chemistry - The Driving Force in Molecular Imaging. Springer, 2006, 289-316
- **L. Cai ; S. Lu ; V. Pike.** *Eur. J. Org. Chem,* 2008, 2853-2873
- **M. Hudlicky.** *Org. React.,* 1988, vol. 35, 513
- **H. Vorbrüggen.** *Synthesis,* 2008, vol. 8, 1165-1174
- **T. Suzuki et al.** *J. Org. Chem.,* 2007, vol. 72, 246-250
- *Journal of Peptide Research,* 2001, vol. 58, 338